# EUROPEAN PATENT SPECIFICATION

(11) **EP 1 932 834 B1**
(45) Date of publication and mention of the grant of the patent: **27.04.2011**
(21) Application number: 06025620.3
(22) Date of filing: 11.12.2006
(51) Int. Cl.: C07D 233/96, C07D 401/14, C07D 407/12, A61K 31/444, A61K 31/505, A61P 35/00

(54) **Aromatic 1,4-DI-Carboxylamides and their use**
Aromatische 1,4-DI-Carboxylamide und deren Verwendung
1,4-DI-carboxylamides aromatiques et leur utilisation

(43) Date of publication of application: 18.06.2008
(73) Proprietor: The Genetics Company, Inc., 4010 Basel (CH)
(72) Inventor: Garcia, Gabriel., Lansdale, PA 19446 (US); Daram, Pierre., 8707 Uetikon am See (CH); Froesch, Barbara., 8932 Mettmenstetten (CH); Lemaillet, Guy., 8706 Meilen (CH); Scapozza, Leonardo., 1274 Grens (CH)
(74) Representative: Stolmár, Matthias

(56) References cited:
- WO-A-02/096430
- WO-A-03/006447

## Description

The present invention relates to aromatic 1,4-Di-Carboxylamides which inhibit the interaction between β-catenin and BCL9 and/or BCL9L proteins and their use for the preparation of a pharmaceutical compositions and for the therapy of certain diseases.

More generally, the present invention relates to inhibitors of the Wnt transduction pathway. Such inhibitors can be used for stem cell research or for the treatment of diseases characterized by aberrant Wnt activation such as cancer, bone and cartilage diseases. Pathological activation of the Wnt pathway has been extensively reported for colorectal cancer, hepatocellular carcinoma, breast cancer, melanomas, mesotheliomas, lymphomas and leukemias. Furthermore, since the Wnt pathway also plays a fundamental role in T-cell development (Staal, Meeldijk et al. 2001; Staal and Clevers 2003), the Wnt signal transduction pathway inhibitors disclosed herein can also be used as immunosuppressant drugs, e.g. after organ transplantation or to treat certain autoimmune diseases such as Lupus erythematosus, multiple sclerosis and rheumatoid arthritis.

Wnt/Wg proteins exert many of their effects on vertebrate animal development by activating the expression of specific target genes in responding cells. Several of these target genes have been identified and some of their functions are consistent with control of cellular growth, differentiation, and survival (He, Sparks et al. 1998; Crawford, Fingleton et al. 1999; Tetsu and McCormick 1999; Kolligs, Nieman et al. 2002; Shtutman, Zhurinsky et al. 2002).

An intricate machinery has been identified which normally marks β-catenin for degradation by phosphorylation. Importantly, the tumor suppressors APC and Axin are essential components of this β-catenin destruction complex. Upon activation of the Wnt pathway, β-catenin escapes this phosphorylation reaction, accumulates in the cytoplasm, and enters the nucleus, where it associates with TCF proteins and the recently identified Lgs/BCL9 [Kramps, T. et al., 2002] proteins to function as a transcriptional coactivator of target genes.

This set-up, in which the key transducer is continuously held in check, is highly susceptible to mutations in its inhibitory components. In fact, mutations in the downstream components of Wnt signalling have been found to be associated with a variety of human cancers (Kinzler and Vogelstein 1996; Miller, Hocking et al. 1999). For example, germline APC mutations can cause hundreds of benign colorectal tumors, some of which develop into cancer. Somatic mutations of the APC gene are associated with 85% of sporadic colorectal adenomas and carcinomas (Kinzler and Vogelstein 1996), mutations in the phosphorylation sites of β-catenin have been found in many human cancers, such as colorectal cancer, hepatocellular carcinoma, and melanoma (Morin, Sparks et al. 1997; Rubinfeld, Robbins et al. 1997) (Caca, Kolligs et al. 1999), and axin mutations have been identified in hepatocellular carcinoma (Satoh, Daigo et al. 2000). Moreover, several mutations and/or changes in expression of upstream components like LRP5, sfrps, WIF-1, DKK or Wnt ligands have not only been linked to cancer, but also to bone and cartilage diseases. Since all these mutations lead to the accumulation of nuclear β-catenin, this protein and its interacting partners has emerged as attractive targets to inhibit Wnt dependent gene expression.

In fact, inhibitors of the Wnt pathway that target the β-Catenin-Tcf4 interaction and processes for finding such inhibitors have been disclosed in various patent applications. International patent application W0 98/42296 discloses purified proteins and conventional processes for screening for inhibitors. International patent application WO 02/44378 describes a conventional process for screening for Tcf-β-Catenin inhibitors. International patent application WO 03/006447 relates to Tcf4-β-Catenin inhibitors. International patent application WO 02/096430 discloses cephalosporine derivatives as small molecule β-catenin inhibitors. International patent application WO 01/19353A2 characterizes a "targetable" pocket in β-Catenin using a 3D-model of the Tcf4-β-Catenin interaction. It also describes *in silico* screening processes based on said interaction for identifying inhibitors as well as a number of inhibitors identified by this process.

The main disadvantage of the above mentioned approach is the nature of the β-Catenin-Tcf4 interaction. In particular, this protein-protein interaction surface is quite large and it is at least partially shared with other β-Catenin interacting partners like ECadherin and APC (Graham, Weaver et al. 2000; Eklof Spink, Fridman et al. 2001; Huber and Weis 2001; Poy, Lepourcelet et al. 2001), which raises serious questions about its specificity. Therefore, alternative targets for the Wnt pathway are required.

The Legless(Lgs)/BCL9 family of proteins was identified in a genetic screening for novel positive regulators of the Wnt pathway downstream of the APC tumor supressor in Drosophila melanogaster. In humans there are two homologues, BCL9 and BCL9-like (BCL9L, also known as B9L or BCL9-2), which, like the Drosophila protein, act as essential adaptor molecules between the transcriptional active components Pygo and β-Catenin. Binding of BCL9 proteins to β-Catenin is essential for the propagation of the Wnt signaling in cancer cells.

Competitor peptides that are capable of disrupting this protein-protein interaction or reduction of BCL9L expression by small interfering RNAs (siRNA) strongly inhibit the Wnt pathway and lead to differentiation of cancer cells from a non-differentiated malignant type into moderately to well differentiated tissue (US2002/0086986). However, despite their specific and potent inhibitory activity on the Wnt pathway these peptides or siRNA molecules do not represent ideal drugs for tumor therapy because of the poor membrane permeability and poor systemic availability.

It is one of the objects of the present invention to provide novel inhibitors of the beta-Catenin/BCL9-BCL9L interaction, in particular compounds with improved drug features, such as lower molecular weight and good cellular permeability.

It was found that compounds with general formula I satisfy these requirement and inhibit efficiently the beta-Catenin/BCL9-BCL9L interaction: 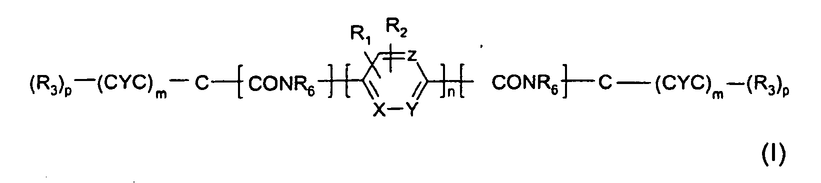 wherein
X, Y, Z represent independently from one another C or N n stands for 1, 2, 3, m is 0 or 1, p stands for 0 or an integer from 1 to 6,
R₁, R₂ represent independently from one another hydrogen, a halogen atom, a hydroxyl group, a C₁-C₃ alkyl group and a C₁-C₃ alkoxy group,
R₃ represents, independently from one another if p is not 0, hydrogen, halogen, a C₁-C₅ linear or branched alkyl, a carboxylyl, a carbomethoxyl, carboethoxyl, a benzyl, an acyl, a hydroxyl, a C₁-C₄ linear or branched alkoxyl, a trifluoromethyl, a cyano, a morpholino, a 1,3-dioxolyl, an N-acetylamidyl or an amidoyl group, a saturated 5-8 membered ring, a heterocyclic ring, optionally substituted by a C₁-C₃ alkyl, a hydroxyl or a benzyl group, a C₁-C₆ alkylsulfonyl, a mono or disubstituted C₁-C₅ alkyl group, a branched or a cyclic amine.
R₆ is H or part of a alicyclic or heteroalicyclic ring system,
with the proviso
if m is 0 then C represents CF₃ or a branched or unbranched C₁-C₄ alkyl group,
if m = 1 then C represents -CH₂-O-, -CH₂-, -CH₂CH₂-, -CH₂CH₂CH₂-, -CH₂(CH₂)CH₂-or denotes a chemical bond between N-C or C-C atoms,
the CONR₆ group may be linked to C either via its carbon or via its nitrogen atom,
CYC stands for a by R₃ substituted or unsubstituted phenyl, pyridinyl, naphthyl, quinolinyl, isoquinolinyl, isoxazolinyl, thiophenyl, 1,3,4-thiadiazazolidinyl, furanyl, tetrahydroquinolinyl, tetrahydroisoquinolinyl, morpholinyl, furanyl, cyclohexenyl and chromen-2-on-yl.

In preferred embodiments of the invention n is 1. The basic cyclic 1,4 substituted structure is therefore a phenyl, pyridinyl, pyridazinyl, pyrazinyl or pyrimidinyl ring.

In still further preferred embodiments of the present invention, n is 2 and the corresponding 1, 8 disubstituted cyclic basic structural motifs are biphenyl, bipyridinyl, bipyridazinyl, bipyrazinyl or bipyrimidinyl.

In further more preferred embodiments n is 3. The corresponding most preferred 1, 12 disubstituted structural motifs consisting of three cyclic basic structures are paraterphenyl, 2,5 diphenylpyridinyl, 3,4 diphenylpyridazinyl and 2,5 diphenylpyrazinyl.

In specific preferred embodiments, X, Y and Z are C, thus phenyl, biphenyl and terphenyl structural motifs form the basic cyclic structure.

In other preferred embodiments, X and Y are C and Z is N. These preferred structural motifs are for example pyridinyl, bipyridinyl and terpyridinyl.

In another preferred embodiment, X is C and Y and Z are N. The corresponding preferred structural motifs are pyridazinyl, pyrimidinyl, pyrazinyl and bipyridazinyl, bipyrazinyl or bipyrimidinyl.

In still further embodiments also mixtures of different structural motifs containing a different number of N atoms are preferred, like 2,5 diphenylpyridinyl, 3,4 diphenylpyridazinyl and 2,5 diphenylpyrazinyl.

In another preferred embodiment, m is 0 and C is a CF₃ group or a branched or unbranched C₁-C₄ alkyl group, specifically, methyl, ethyl, n-propyl, isopropyl, n-butyl, i-butyl, tert-butyl and isopropyl. In an especially preferred embodiment, C is tert-butyl or isopropyl.

In further preferred embodiments of the invention, m is 1. In this case, C is selected from the group consisting of -CH₂-O-, -CH₂-, -CH₂CH₂-, -CH₂CH₂CH₂-, - CH₂(CH₂)CH₂- or denotes a chemical bond between N-C or C-C atoms. In most preferred embodiments, C denotes a chemical bond between N-C or C-C atoms.

In one further specifically preferred embodiment, the CONR₆ group in formula I is linked to C by its carbon atom. In an alternative embodiment which is equally preferred, the CONR₆ group is linked to C by its nitrogen atom. Thereby a fine tuning for preferred embodiments for C with respect to steric factors, acidity, further substitution etc. is provided.

In the context of the present invention, "halogen" means Cl, Br, I and F. Especially preferred are Cl, Br and F, more preferred Cl and Br.

A specifically preferred compound according to the invention has the following formula:

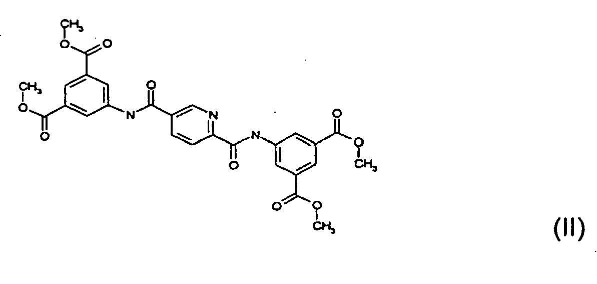

A further specifically preferred compound according to the invention has the following formula:

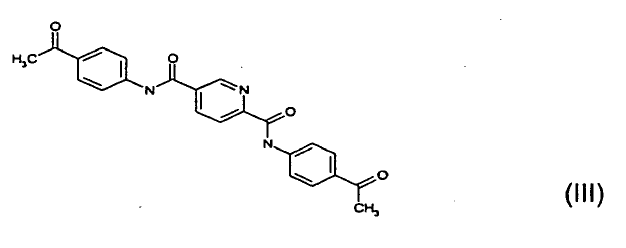

A still further specifically preferred compound according to the invention has the following formula:

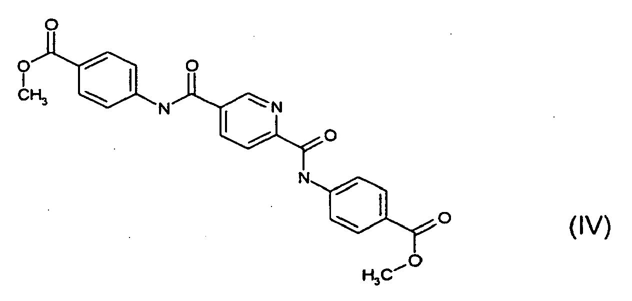

Still further preferred compounds have the following formulae

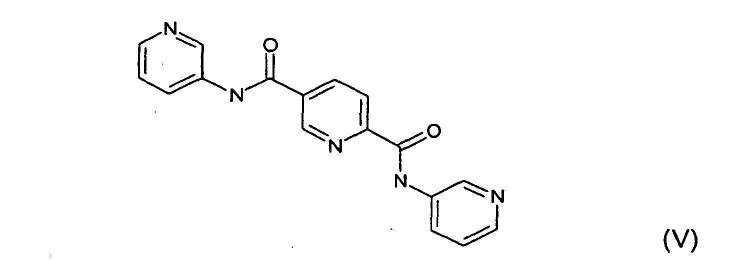

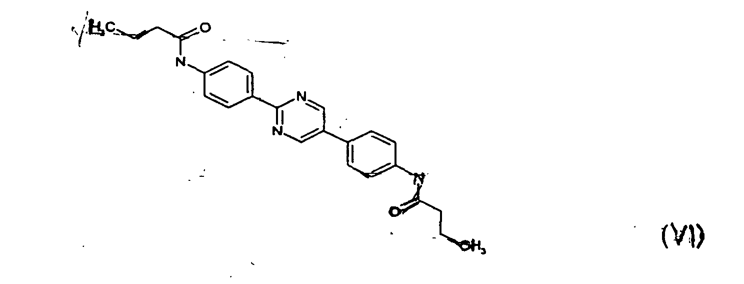

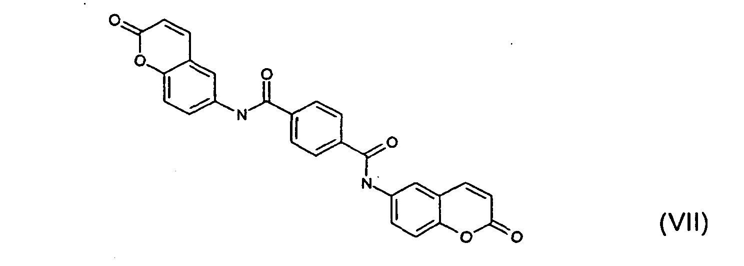

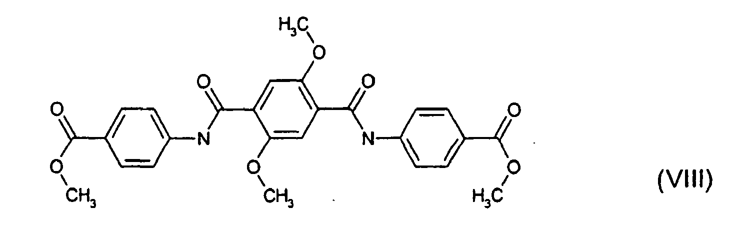

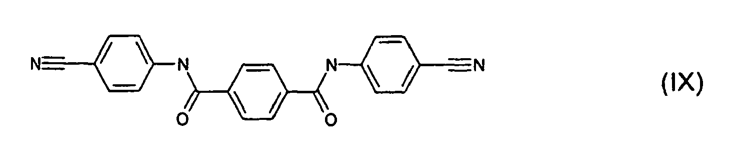

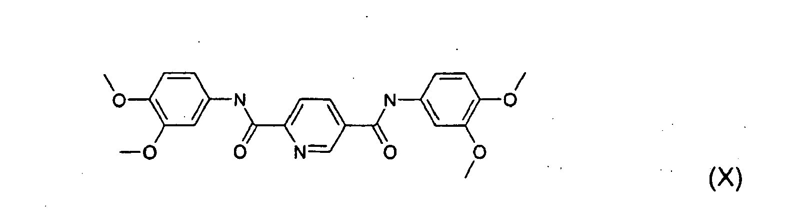

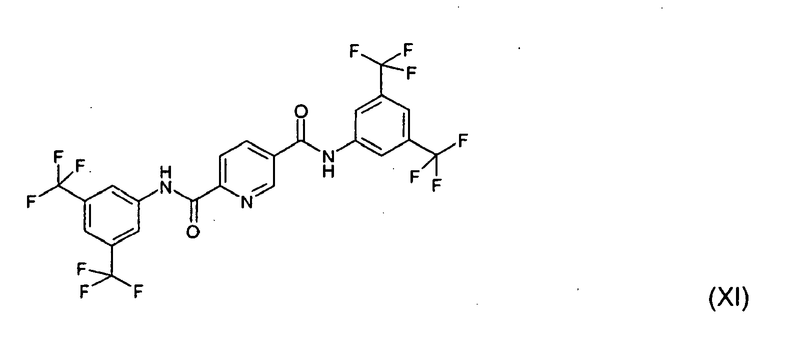

As shown in the preferred examples one of the most characteristic features is the "linear" 1,4, 1,8 or 1,12 substitution pattern at the "central" aromatic core. This is especially advantageous since the epitope, the binding pocket for the interaction with the peptide is a straight, channel-like ("linear") structure, so that the compounds according to the present invention fit sterically particularly well into the pocket and enable the specificity for binding with the key residues of the epitope.

A further object of the invention is the use of a compound according to the invention as a medicament and for the manufacture of a pharmaceutical composition for the treatment of diseases characterized by the over-activation of the Wnt pathway, such as, but not limited to, colon cancer, breast and prostate cancer, lung cancer, hepatocarcinoma, ovarian cancer, melanoma, and bone and join diseases like osteo- or rheumatoid-arthritis

The invention relates further to a pharmaceutical composition comprising a compound according the invention or a pharmaceutically acceptable salt thereof and in still further embodiments further comprising at least one pharmaceutically acceptable carrier, diluent or excipient.

### Figures

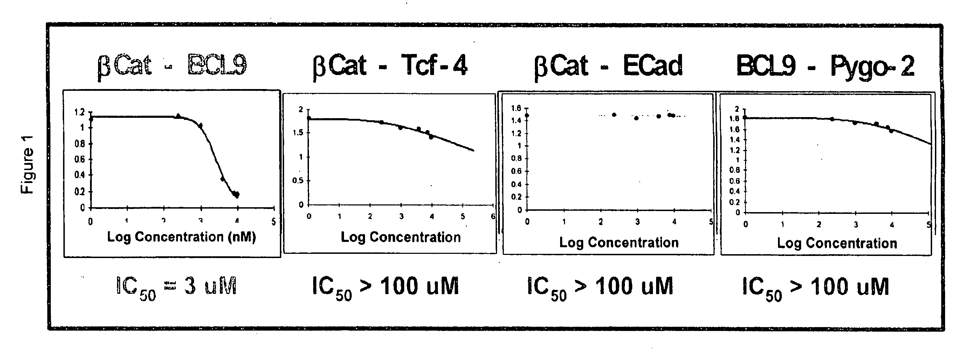

Figure 1. shows the results of a compound according to the invention (TGC0012296) when tested for inhibition of the BCL9/BCL9L-β-Catenin, Tcf-4-β-Catenin and ECadherin-β-Catenin interactions as described in Example 1 (ELISA assay). The x-axis shows the concentration as the logarithmic function (log) in nM and the y-axis the measured absorbance.

The following examples and specifically indicated compounds further illustrate the best mode contemplated by the inventors for carrying out their invention. The examples relate to preferred embodiments and are not to be construed to be limiting on the scope of the invention.

### Examples

### Example 1: In vitro ELISA-based protein-protein interaction assay

### Protein production in bacteria

cDNAs including the binding domains of Lgs and beta-Catenin were fused downstream of glutathione-S-transferase (GST) cDNA in pGEX-4T inducible bacterial expression vector (Pharmacia). Similar recombinant DNA constructs were generated in the pET-32a(+) vector (Novagen) for expression of fusion protein containing the Thioredoxine-6His tag at their N-terminus. Fusion proteins were produced in BL21 bacteria (e.g. Stratagene) following manufacturer's recommendation.

To isolate the produced fusions protein, bacteria were ground in aluminum oxide (Sigma, A2039) and the resulting powder extracted in lysis buffer (140mM NaCl, 10mM Na2HP04, 1.BmM KH2P04, 2.7mM KCI, 1mM EDTA, 1% Triton-X-100, 1mM DTT and protease inhibitors)under rotation for 30 minutes. Supernatants were cleared by centrifugation then filtered on a 0.45µm membrane before protein purification. For GST tagged protein, lysate were processed on glutathione agarose beads following manufacturer's recommendation (Amersham Pharmacia) and purified proteins eluted in 50mM Tris-Cl, 10mM reduced glutathione pH8.0. For thioredoxine-6His fusions, tagged proteins were purified from the lysate on a nickel chelating column (e.g. Hitrap chelating, G.E. Healthcare) using an HPLC system (e.g. Akta Prime, G.E. Healthcare) and eluted under increasing concentration of imidazole. The purified proteins solution was dialyzed against PBS before use. The quality of the preparations was checked by SDS-gel electrophoresis by standard methods well known by persons skilled in the art.

Compound preparation: Compounds can be prepared as 25 mM stock in DMSO and diluted in DMSO to reach final test concentrations of up to 100µM. Final DMSO concentration in the assay is 5%.

### ELISA-Assay

100µl of GST fusion protein (*e.g*. GST-βCatenin) diluted to the appropriate concentration in phosphate buffer saline are added per well to a 96 well plate with high protein binding capacity (*e.g*. Nunc Maxisorp) and placed at 4°C overnight. The following day the liquid in the wells is discarded and 150µl 3% bovine serum albumin (BSA) in PBS are added to each well and incubated at room temperature for 2 hours under moderate shaking. This solution is then discarded and each well washed twice with 200µl PBS. After carefully discarding the second wash, a mixture of 5µl compound dilution and 95µl 6His fusion protein (e.g. 6His-Bcl9) diluted to the appropriate concentration in PBS is added to each well. The plate is then incubated for 1 hour at room temperature under moderate shaking. This protein solution is then discarded and the wells washed 3 times with 200µl 0.1 % Tween-20 solution in PBS. After carefully discarding the final wash, 100µl of anti 6His antibody conjugated to the horseradish peroxidase (e.g.Roche antibody 11965085001) at the appropriate dilution in 1% BSA in PBS is added to the wells and the plate incubated for 1 hour at room temperature under moderate shaking. This solution is then discarded and the wells washed 3 times with 200µl 0.1 % Tween-20 solution in PBS. After carefully discarding the final wash, 100µl of HRP substrate Tetramethylbenzamidine (TMB) in solution (*e.g*. Sigma T0440) is added to the wells and the plate incubated up to 30 minutes in the dark at room temperature. The reaction is stopped by addition of 100µl of 2N HCl to each well and the optical density is measured at 450 nm and 630 nm.

### Example 2: Inhibition of Wnt activity in cancer cells

The effect of β-catenin/BCL9 inhibitors identified in the ELISA-based protein-protein interaction assay on the Wnt pathway can be evaluated in a cell culture system using a reporter gene responsive to the Tcf/Lef family of transcription factors or by quantitative analysis of Wnt target genes in appropriate cell lines.

A reporter gene is a construct which comprises a readily detectable or assayable gene such β-galactosidase, green fluorescent protein, chloramphenicol acetyltransferase or luciferase, linked *in cis* to a transcription factor response element and a minimal promoter. Depending on the expression vectors used, this protocol can be applied, e.g. for mammalian as well as for *Drosophila* cell lines. For instance, colon cancer cells with constitutively active Wnt pathway like SW620 (ATCC) are a well suitable system. Hereby, a Tcf-4 driven luciferase reporter plasmid (i.e. TOPFLASH, Upstate biotechnology, New York, USA) is transiently or stably transfected into cells known in the art. Any means for introducing genetic material into cells can be used, including but not limited to infection, electroporation or transfection. For instance, to introduce DNA into SW620 cells, a lipofection agent like the Lipofectamine transfection reagent (Life Technologies, Inc.) can be used. By transient transfection protocols, a second reporter gene, e.g. the renilla luciferase reporter plasmid pRL-SV40 (Promega Corporation, Madison USA), needs to be co-transfected to normalize for transfection efficiency. Drugs are added to the media 24h after transfection (transient transfection) or 24h after seeding of the cells (stably transfected cells). Cell extracts are prepared 24 to 48 h later and assayed for reporter gene activity as described by the manufacturer (eg. for luciferase activity: Promega Corporation). Compounds reducing reporter gene activity more than 50% compared to solvent alone treated cells are considered as hits. In parallel, toxicity can be assessed for instance by the yellow tetrazolium salt cell proliferation assay (MTT) assay.

Table 1 shows the *in vitro* activities of specific compounds according to the invention. TGC ID refers to the applicants internal identification number; Specificity values depict compound's specificity for inhibition of the beta-Catenin-BCL9-BCL9L (specific means that they do not significantly inhibit 1E-Cadherin-βCatenin, Tcf-4-βCatenin or BCL9-Pygo interactions); MW means molecular weight.

**Table 1. Compound activities (IC50 in µM) as measured by the ELISA based protein-protein interaction assay as described in Example 1**

| **MOLSTRUCTURE** | **TGC ID** | **A-LOGP** | **PSA** | **MW** | β**Cat- BCL 9** | β**Cat- TCF4 TCF4** | β**Cat-ECad** |
|---|---|---|---|---|---|---|---|
| 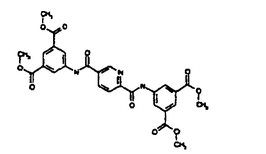 | TGC0000160 | 2.73 | 176 | 549 | 38 | > 100 | |
| | TGC0000161 | 10.11 | 129 | 715 | > 50 | | |
| 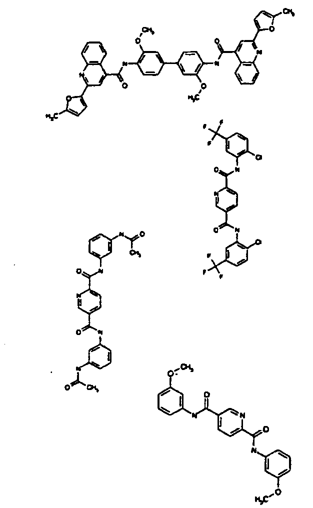 | TGC0011278 | 6.93 | 71 | 522 | > 100 | | |
| | TGC0011279 | 3.5 | 129 | 431 | > 100 | | |
| | TGC0011280 | 3.6 | 90 | 377 | > 100 | 23 | 21 |
| 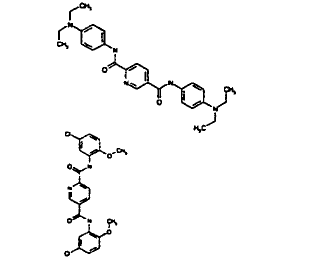 | TGC0011281 | 5.28 | 78 | 460 | > 100 | | |
| | TGC0011282 | 4.91 | 90 | 446 | > 100 | | |
| 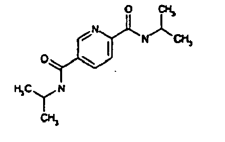 | TGC0011283 | 1.36 | 71 | 249 | > 100 | | |
| 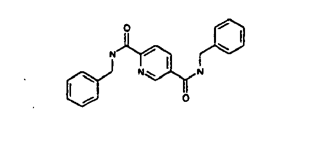 | TGC0011284 | 2.94 | 71 | 345 | > 100 | | |
| 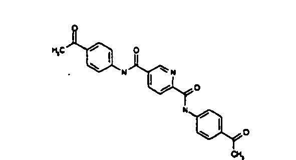 | TGC0011285 | 3.99 | 105 | 401 | 6 | >10 | >10 |
| 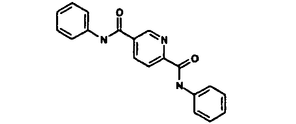 | TGC0011286 | 3.59 | 71 | 317 | > 100 | | |
| 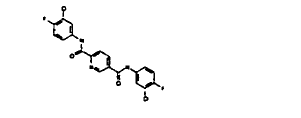 | TGC0011287 | 5.17 | 71 | 422 | > 100 | | |
| 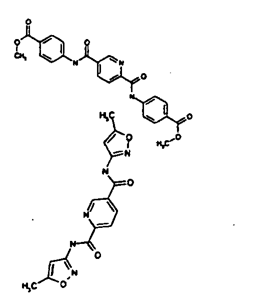 | TGC0011288 | 3.16 | 124 | 433 | 2 | >10 | >10 |
| | TGC0011289 | 2.18 | 123 | 327 | > 50 | | |
| 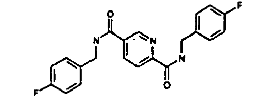 | TGC0011337 | 3.22 | 71 | 381 | >10 | | |
| 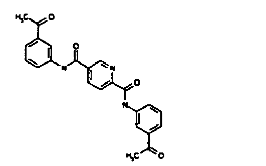 | TGC0011338 | 3.99 | 105 | 401 | > 10 | | |
| 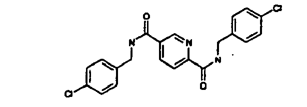 | TGC0011339 | 4.25 | 71 | 414 | > 10 | | |
| 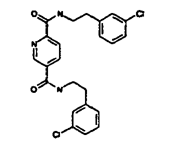 | TGC0011340 | 4.33 | 71 | 442 | > 20 | | |
| 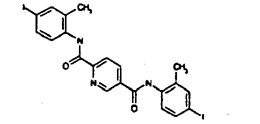 | TGC0011341 | 5.41 | 71 | 597 | > 10 | | |
| 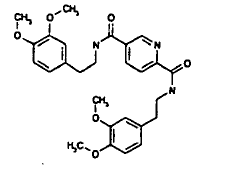 | TGC0011342 | 3.06 | 108 | 494 | >50 | >50 | >50 |
| 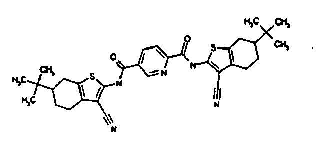 | TGC0011343 | 7.75 | 175 | 600 | > 100 | | |
| 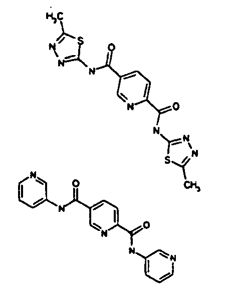 | TGC0011344 | 1.91 | 179 | 361 | 86 | 119.2 | 41.4 |
| | TGC0011423 | 2.38 | 90 | 319 | 29 | > 100 | > 100 |
| 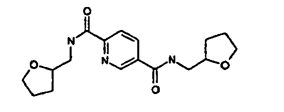 | TGC0011424 | 0.9 | 90 | 333 | > 50 | | |
| 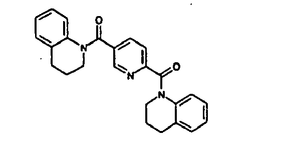 | TGC0011425 | 4.27 | 54 | 397 | > 25 | | |
| 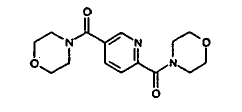 | TGC0011426 | 0.03 | 72 | 305 | > 50 | | |
| 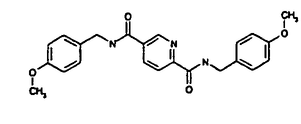 | TGC0011427 | 2.96 | 90 | 405 | > 25 | | |
| 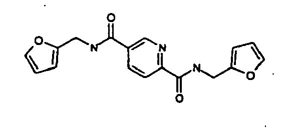 | TGC0011428 | 2.13 | 97 | 325 | > 50 | > 50 | > 50 |
| 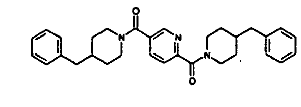 | TGC0011429 | 5.27 | 54 | 482 | > 25 | | |
| 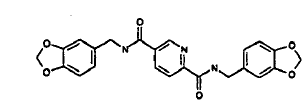 | TGC0011430 | 2.4 | 108 | 433 | > 50 | > 50 | > 50 |
| 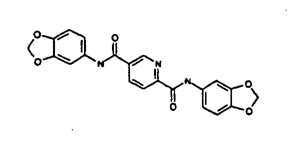 | TGC0011431 | 3.04 | 108 | 405 | > 50 | | |
| 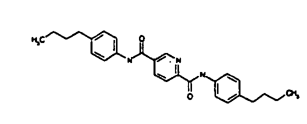 | TGC0011433 | 6.27 | 71 | 430 | > 50 | | |
| 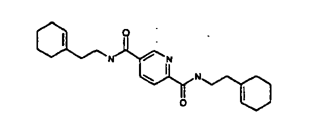 | TGC0011435 | 4.32 | 71 | 382 | > 50 | | |
| 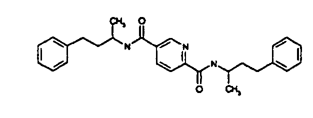 | TGC0011436 | 4.58 | 71 | 430 | > 10 | | |
| 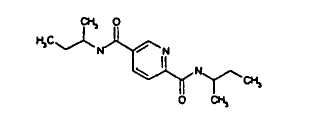 | TGC0011437 | 2.14 | 71 | 277 | > 50 | | |
| 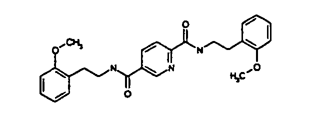 | TGC0011445 | 3.04 | 90 | 434 | > 10 | | |
| 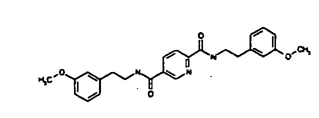 | TGC0011446 | 3.04 | 90 | 434 | > 10 | | |
| 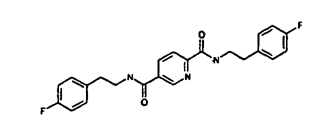 | TGC0011447 | 3.3 | 71 | 409 | > 50 | | |
| 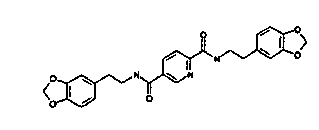 | TGC0011448 | 2.48 | 108 | 461 | > 10 | | |
| 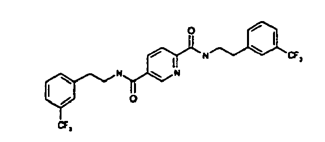 | TGC0011452 | 4.82 | 71 | 511 | > 10 | | |
| 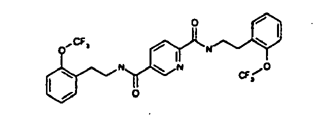 | TGC0011454 | 5.07 | 90 | 543 | > 100 | | |
| 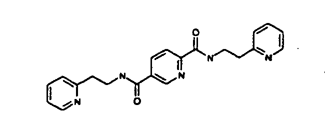 | TGC0011455 | 1.82 | 97 | 375 | > 100 | | |
| 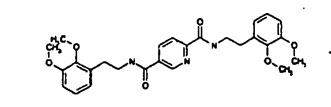 | TGC0011456 | 3.06 | 108 | 494 | > 100 | | |
| 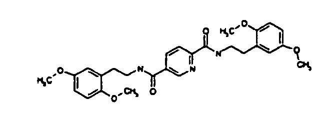 | TGC0011457 | 3.06 | 108 | 494 | > 100 | | |
| 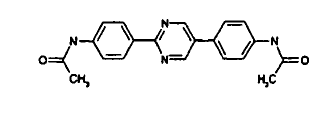 | TGC0011524 | 3.73 | 84 | 346 | > 100 | | |
| 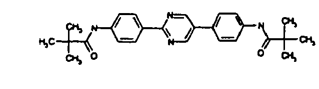 | TGC0011525 | 5.78 | 84 | 431 | > 10 | | |
| | TGC0011526 | 4.81 | 84 | 454 | > 100 | | |
| 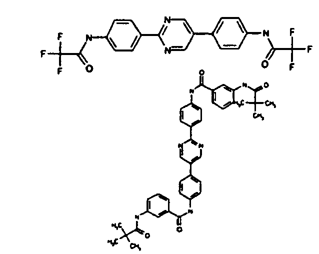 | TGC0011527 | 8.28 | 142 | 669 | > 10 | | |
| 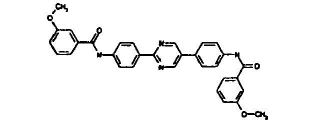 | TGC0011528 | 6.33 | 102 | 531 | > 50 | > 50 | > 50 |
| 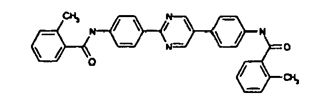 | TGC0011529 | 6.93 | 84 | 499 | > 100 | | |
| 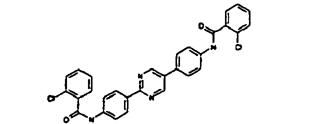 | TGC0011530 | 7.62 | 84 | 539 | >10 | | |
| 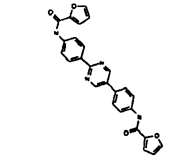 | TGC0011531 | 5.5 | 110 | 450 | >10 | | |
| | TGC0011533 | 7.84 | 84 | 628 | > 100 | | |
| 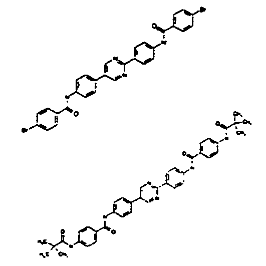 | TGC0011534 | 8.28 | 142 | 669 | > 100 | | |
| 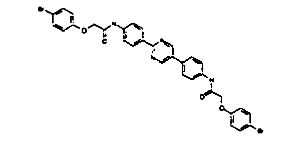 | TGC0011565 | 7.37 | 102 | 688 | > 100 | | |
| 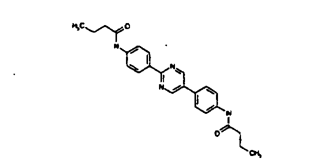 | TGC0011566 | 5.29 | 84 | 403 | 68 | > 100 | > 100 |
| 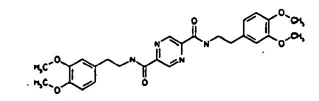 | TGC0011592 | 2.46 | 121 | 495 | > 10 | | |
| 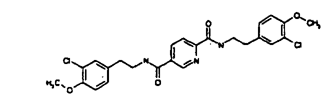 | TGC0011593 | 4.35 | 90 | 502 | > 100 | | |
| 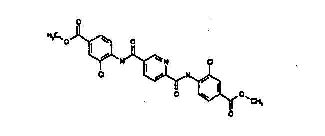 | TGC0011605 | 4.47 | 124 | 502 | > 50 | 22 | 21 |
| 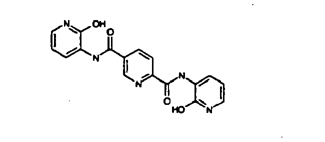 | TGC0012261 | 1.79 | 137 | 351 | > 100 | | |
| 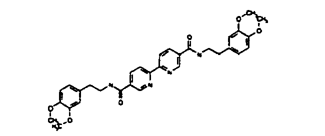 | TGC0012281 | 4.12 | 121 | 571 | 38 | | |
| 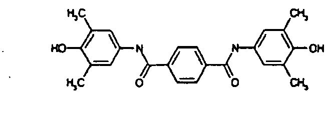 | TGC0012287 | 4.84 | 99 | 404 | n.d. | | |
| 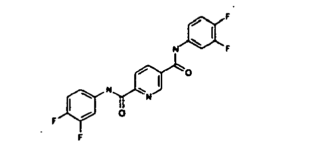 | TGC0012289 | 4.14 | 71 | 389 | > 50 | | |
| 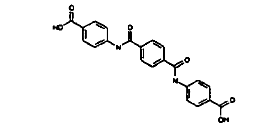 | TGC0012290 | 0.92 | 138 | 404 | > 50 | | |
| 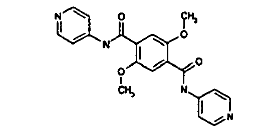 | TGC0012291 | 3 | 102 | 378 | > 50 | | |
| 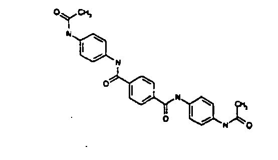 | TGC0012292 | 4.11 | 116 | 430 | > 50 | | |
| 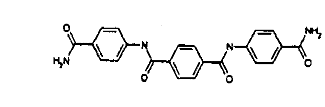 | TGC0012293 | 2.39 | 144 | 402 | > 10 | | |
| 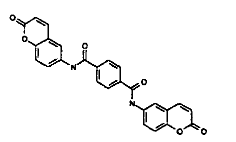 | TGC0012296 | 4.4 | 119 | 452 | 3 | >10 | >10 |
| 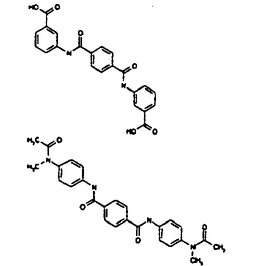 | TGC0012297 | 0.92 | 138 | 404 | > 100 | | |
| | TGC0012298 | 4.16 | 99 | 459 | n.d. | | |
| 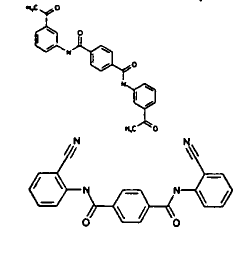 | TGC0012299 | 4.6 | 92 | 400 | n.d. | | |
| | TGC0012300 | 3.93 | 106 | 366 | > 10 | | |
| 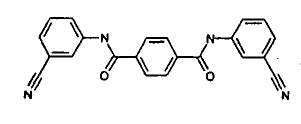 | TGC0012301 | 3.93 | 106 | 366 | > 50 | | |
| 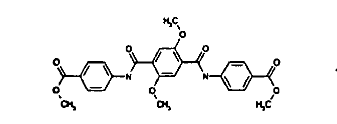 | TGC0012302 | 3.78 | 129 | 492 | 94 | >100 | > 100 |
| 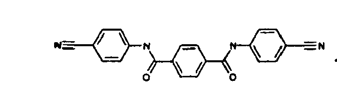 | TGC0012303 | 3.93 | 106 | 366 | 5 | >10 | >10 |
| 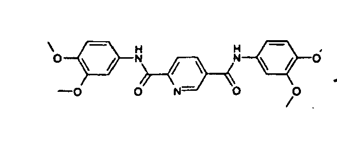 | TGC0012373 | 3.62 | 108 | 437.5 | 10 | | |
| 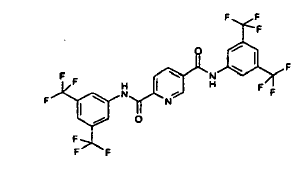 | TGC0012379 | 7.66 | 71 | 589 | 20 | | |

As is evident from the table, the compounds according to formulae II to XI show especially advantageous results in terms of reactivity and specificity towards the beta-Catenin/BCL9-BCL9L interaction.

The components according to the invention can be prepared as follows using a general synthetic pathway.

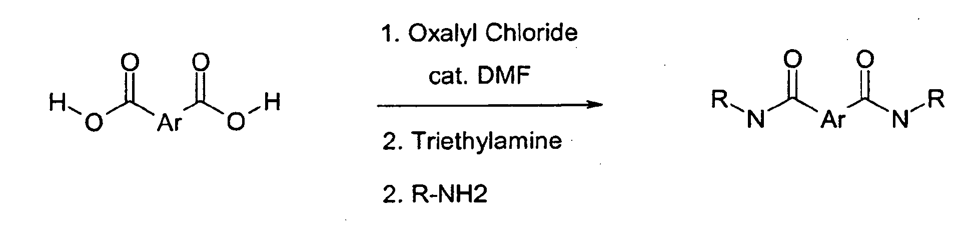

Dicarboxylic acid (1 eq.) was suspended in dichloromethane (5 mL/mmol of acid) and cooled to 0°C. Oxalyl chloride (2.2 eq.) was then syringed in followed by the addition of 4 drops of DMF to get the reaction started. The reaction mixture was allowed to react at 0°C to room temperature overnight under vigorous stirring.

Excess oxalyl chloride was then removed and the remaining reaction mixture cooled to 0°C. A solution of triethylamine (3 eq.) in dichloromethane (5 mL/mmol of triethylamine) was syringed in followed by the slow addition of a solution of the corresponding aniline (3 eq.) in dichloromethane (5 mL/mmol of aniline). The resulting reaction mixture was allowed to stir overnight while allowing the temperature to go from 0°C to room temperature. The reaction mixture was filtered then washed with water. Upon evaporation of the solvent, the crude product was either crystallized from methanol or column chromatographed to a white powder.

Selected Mass Spectral (MS) and 1 H-NMR data of exemplary compounds according to the invention are given in the following table 2 (signals given in ppm in reference to deuterated dimethylsulfoxide, s: singlet, d: doblet, t: triplet, q: quartet, dd: doublet of doublets, dt: doublet of triplets, m: multiplet and b: broad band):

**Table 2: Selected Mass Spectral and 1H-NMR data of compounds according to the invention**

| **MOLSTRUCTURE** | **TGC ID** | **MS** | **1H-NMR** |
|---|---|---|---|
| | TGC0000160 | 549 | 3.93 (s, 12 H), 8.37 (s, 2H), 8.40 (d, 1 H), 8.67(d, 1 H), 8.79 (s, 2H), 8.94 (s, 2H), 9.32 (s, 1 H), 11.11 (s, 1H), 11.39 (s, 1 H). |
| 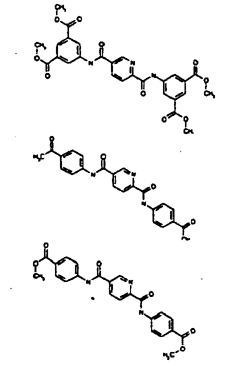 | TGC0011285 | 400 | 2.04 (s, 6H), 7.58 (dd, 4H), 7.69 (d, 2H), 7.85 (d, 2H), 8.27 (d, 1H), 8.54 (d, 1H), 9.19 (s, 1H), 9.97 (s, 2H), 10.30 (b, 1H), 10.71 (s, 1 H). |
| | TGC0011288 | 432 | 4.02 (s, 6H), 7.99 (m, 4H), (8.10 (m, 2H), 8.27 (dd, 1 H), 8.53 (dd, 1 H), 9.18 (d, 1 H), 10.88 (s, 1 H), 11.09 (s, 1 H). |
| 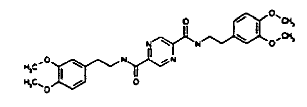 | TGC0011592 | 494 | 2.90 (t, 4H), 3.74 (t, 4H), 3.87 (s, 6H), 3.88 (s, 3H), 6.85-6.77 (m, 6H), 7.92 (t, 2H), 9.28 (s, 2H). |
| 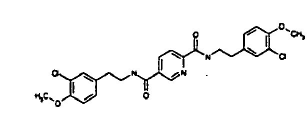 | TGC0011593 | 502 | 2.87 (t, 4H), 3.70 (t, 4H), 3.89 (s, 6H), 6.22 (t, 1 H), 6.90-6.86 (m, 2H), 7.08 (t, 1 H), 7.10 (t, 1H), 7.26 (t, 2H), 8.23-8-09 (m, 2H), 8.82 (d, 1 H), 8.83 (s, 1H). |
| 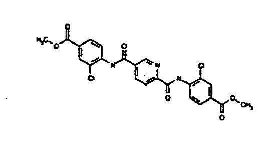 | TGC0011605 | N.A. | 3.88 (s, 6H), 7.99 (d, 2H), 8.01 (s, 1H), 8.32 (d, 2H), 8.56 (t, 3H), 9.18 (s, 1 H), 10.80 (s, 2H). |
| 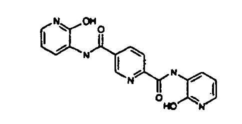 | TGC0012261 | 349 | 7.43 (dt, 5H), 7.67 (d, 2H), 7.94 (d, 2H), 14.3-13.5 (b, 2H). |
| 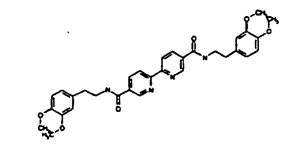 | TGC0012281 | N.A. | 3.00 (t, 4H), 3.71 (q, 4H), 3.83 (s, 6H), 3.85 (s, 6H), 6.78 (t, 3H), 6.84 (s, 3H), 8.05 (q, 4H), 9.19 (t, 2H), 9.24 (s, 2H). |
| 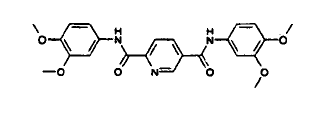 | TGC0012373 | N.A. | 3.76 (s, 6H), 3.77 (s, 6H), 6.97 (dt, 2H), 7.34 (d, 1H), 7.49 (s, 1 H), 7.54 (d, 1H), 7.65 (s, 1H), 8.27 (d, 1 H), 8.55 (d, 1 H), 9.18 (s, 1H), 10.52 (bs, 1 H), 10.62 (s, 1 H). |
| 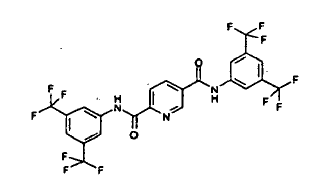 | TGC0012379 | N.A. | 7.87 (d, 2H), 8.37 (d, 1 H), 8.52 (s, 2H), 8.62 (d, 1H), 8.75 (s, 2H), 9.27 (s, 1 H), 11.28 (bs, 1 H), 11.52 (s, 1 H). |

The compounds according to the invention may be administered alone or in the form of a pharmaceutically acceptable salt thereof. A pharmaceutical composition comprising a compound according to the invention or a pharmaceutically active salt thereof may further comprise at least one pharmaceutically acceptable carrier, diluent or excipient. It is understood that in specific embodiments also further active compounds are contained within the composition.

The compounds according to the invention may be formulated for topical, oral, transdermal, parenteral, sublingual, intranasal, intrathecal, rectal, inhalative or intravenous administration in form of e.g. tablets, gel, capsules, patches, ointments, creams. Parenteral delivery can be carried out by depot, syringe, ampoule or vial.

The compounds of the invention, together with a conventional adjuvant, carrier, or diluent, may thus be placed into the form of pharmaceutical compositions and unit dosages thereof, and in such form may be employed as solids, liquids or in the form of sterile injectable solutions. If a solid carrier is used, the preparation may be ta-bleted, placed in a hard gelatine capsule in powder or pellet form, or in form of a troche or lozenge. The solid carrier may contain conventional excipients such as binding agents, tableting lubricants, fillers, disintegrants, wetting agents and the like. Tablets may be film coated by conventional techniques. If a liquid carrier is employed, the preparation may be in form of syrup, emulsion, soft gelatine capsule, sterile vehicle for injection, an aqueous or non-aqueous liquid suspension, or may be a dry product for reconstitution with water or other suitable vehicles before use. Liquid preparations may contain conventional additives such as suspending agents, emulsifying agents, wetting agents, non-aqueous vehicle (including edible oils), preservatives, as well as flavouring and /or colouring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. Administration, however, can also be carried out rectally, e.g., in the form of suppositories, or vaginally, e.g. in the form of pessaries, tampons, creams, or percutaneously, e.g., in the form of ointments, creams or tinctures.

A suitable dose of compounds or pharmaceutical compositions according to the invention for a mammal, especially humans, suffering from, or likely to suffer from any condition as described herein is an amount of active ingredient from about 0.1 µg/kg to 500mg/kg body weight. For parenteral administration, the dose may be in the range of 0.1 µg/kg to 100mg/kg body weight for intravenous administration. The active ingredient will preferably be administered in equal doses from one to four times daily. The compounds of Formula (I) can also be used in the form of a precursor (prodrug) or a suitably modified form that releases the active compound *in vivo.* Normally, the administered dose will be gradually increased until the optimal effective dosage for the treated host is determined. The optimal administered dosage will be determined by a physician or others skilled in the art, depending on the relevant circumstances including the condition to be treated, the choice of compound to be administered, the route of administration, the sex, age, weight, and the specific response of the treated individual in respect to the severity of the individual's symptoms.

The pharmaceutical compositions are prepared by conventional techniques appropriate to the desired preparation containing appropriate amounts of the active ingredient, i.e., the compounds of the present invention. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

### References

Bartel, P. L. and S. Fields (1995). "Analyzing protein-protein interactions using two-hybrid system." Methods Enzymol 254: 241-63.
Boute, N., R. Jockers, et al. (2002). "The use of resonance energy transfer in high-throughput screening: BRET versus FRET." Trends Pharmacol Sci 23(8): 351-4.
Caca, K.; F. T. Kolligs, et al. (1999). "Beta- and gamma-catenin mutations, but not Ecadherin inactivation, underlie T-cell factor/lymphoid enhancer factor transcriptional deregulation in gastric and pancreatic cancer." Cell Growth Differ 10(6): 369-76.
Crawford, H. C., B. M. Fingleton, et al. (1999). "The metalloproteinase matrilysin is a target of beta-catenin transactivation in intestinal tumors." Oncogene 18(18): 2883-91.
Eklof Spink, K., S. G. Fridman, et al. (2001). "Molecular mechanisms of beta-catenin recognition by adenomatous polyposis coli revealed by the structure of an APC-beta-catenin complex." Embo J 20(22): 6203-12.
Gonzalez, J. E. and P. A. Negulescu (1998). "Intracellular detection assays for high-throughput screening." Curr Opin Biotechnol 9(6): 624-31.
Graham, T. A., C. Weaver, et al. (2000). "Crystal structure of a beta-catenin/Tcf complex." Cell 103(6): 885-96.
He, T. C.. A. B. Sparks, et al. (1998). "Identification of c-MYC as a target of the APC pathway [see comments]." Science 281(5382): 1509-12.
Huber, A. H. and W. I. Weis (2001). "The structure of the beta-catenin/E-cadherin complex and the molecular basis of diverse ligand recognition by beta-catenin." Cell 105(3): 391-402.
Kinzler, K. W. and B. Vogelstein (1996). "Lessons from hereditary colorectal cancer." Cell 87(2): 159-70.
Kolligs, F. T., M. T. Nieman, et al. (2002). "ITF-2, a downstream target of the Wnt/TCF pathway, is activated in human cancers with beta-catenin defects and promotes neoplastic transformation." Cancer Cell 1(2): 145-55.
Kramps, T., O. Peter, et al. (2002). "Wnt/wingless signaling requires BCL9/legless-mediated recruitment of pygopus to the nuclear beta-catenin-TCF complex." Cell 109(1): 47-60.
Miller, J. R., A. M. Hocking, et al. (1999). "Mechanism and function of signal transduction by the Wnt/beta-catenin and Wnt/Ca2+ pathways." Oncogene 18(55): 7860-72.
Morin, P. J., A. B. Sparks, et al. (1997). "Activation of beta-catenin-Tcf signaling in colon cancer by mutations in beta-catenin or APC [see comments]." Science 275(5307): 1787-90.
Nasir, M. S. and M. E. Jolley (1999). "Fluorescence polarization: an analytical tool for immunoassay and drug discovery." Comb Chem High Throughput Screen 2(4): 177-90.
Poy, F., M. Lepourcelet, et al. (2001). "Structure of a human Tcf4-beta-catenin complex." Nat Struct Biol 8(12): 1053-7.
Rubinfeld, B., P. Robbins, et al. (1997). "Stabilization of beta-catenin by genetic defects in melanoma cell lines." Science 275(5307): 1790-2.
Satoh, S., Y. Daigo, et al. (2000). "AXIN1 mutations in hepatocellular carcinomas, and growth suppression in cancer cells by virus-mediated transfer of AXIN1." Nat Genet 24(3): 245-50.
Shtutman, M., J. Zhurinsky, et al. (2002). "PML is a target gene of beta-catenin and plakoglobin, and coactivates beta-catenin-mediated transcription." Cancer Res 62(20): 5947-54.
Staal, F. J. and H. C. Clevers (2003). "Wnt signaling in the thymus." Curr Opin Immunol 15(2): 204-8.
Staal, F. J., J. Meeldijk, et al. (2001). "Wnt signaling is required for thymocyte development and activates Tcf-1 mediated transcription." Eur J Immunol 31(1): 285-93.
Tetsu, O. and F. McCormick (1999). "Beta-catenin regulates expression of cyclin D1 in colon carcinoma cells." Nature 398(6726): 422-6.
Topcu, Z. and K. L. Borden (2000). "The yeast two-hybrid system and its pharmaceutical significance." Pharm Res 17(9): 1049-55.
van de Wetering, M., R. Cavallo, et al. (1997). "Armadillo coactivates transcription driven by the product of the Drosophila segment polarity gene dTCF." Cell 88(6): 789-99.

## Claims

1. A compound of formula I 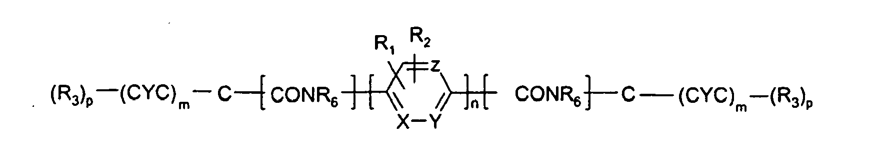 wherein
X, Y, Z represent independently from one another C or N
n stands for 1, 2, 3, m is 0 or 1, p stands for 0 or an integer from 1 to 6,
R₁, R₂ represent independently from one another hydrogen, a halogen atom, a hydroxyl group, a C₁-C₃ alkyl group and a C₁-C₃ alkoxyl group,
R₃ represents, independently from one another if p is not 0, hydrogen, halogen, a C₁-C₅ linear or branched alkyl, a carboxylyl, a carbomethoxyl, carboethoxyl, a benzyl, an acyl, a hydroxyl, a C₁-C₄ linear or branched alkoxyl, a trifluoromethyl, a cyano, a morpholino, a 1,3-dioxolyl, an N-acetylamidyl or an amidoyl group, a saturated 5-8 membered ring, a heterocyclic ring, optionally substituted by a C₁-C₃ alkyl, a hydroxyl or a benzyl group, a C₁-C₆ alkylsulfonyl, a mono or disubstituted C₁-C₅ alkyl group, a branched or a cyclic amine.
R₆ is H or part of a alicyclic or heteroalicyclic ring system,
with the proviso
if m is 0 then C represents CF₃ or a branched or unbranched C₁-C₄ alkyl group,
if m = 1 then C represents -CH₂-O-, -CH₂-, -CH₂CH₂-, -CH₂CH₂CH₂-, - CH₂(CH₂)CH₂- or denotes a chemical bond between N-C or C-C atoms,
the CONR₆ group may be linked to C either via its carbon or via its nitrogen atom, CYC stands for a by R₃ substituted or unsubstituted phenyl, pyridinyl, naphthyl, quinolinyl, isoquinolinyl, isoxaxolinyl, thiophenyl, 1,3,4-thiadiazazolidinyl, furanyl, tetrahydroquinolinyl, tetrahydroisoquinolinyl, morpholinyl, furanyl, cyclohexenyl and chromen-2-on-yl.

2. A compound according to claim 1, wherein n is 1

3. A compound according to claim 1, wherein n is 2

4. A compound according to claim 1, wherein n is 3

5. A compound according to any of claims 1 to 4, wherein X, Y and Z are C.

6. A compound according to any of claims 1 to 4, wherein X and Y are C and Z is N.

7. A compound according to any of claims 1 to 4, wherein X is C and Y and Z are N.

8. A compound according to any of claims 1 to 7, wherein m is 0.

9. A compound according to claim 8, wherein C is CF₃ or a branched or unbranched C₁-C₄ alkyl group.

10. Compound according to claim 9, wherein C is tert-butyl.

11. A compound according to any of claims 1 to 7, wherein m is 1

12. A compound according to claim 11, wherein C is -CH₂-O-, -CH₂-, -CH₂CH₂-, -CH₂CH₂CH₂-, -CH₂(CH₂)CH₂- or denotes a chemical bond between N-C or C-C atoms.

13. A compound according to any of claims 1 to 12, wherein the CONR₆ group is linked to C by its carbon atom.

14. A compound according to any of claims 1 to 12, wherein the CONR₆ group is linked to C by its nitrogen atom.

15. A compound according to claim 1 with formula II:

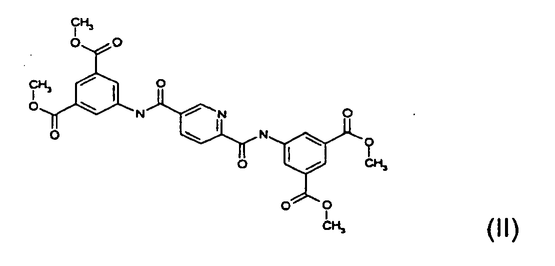

16. A compound according to claim 1 with formula III:

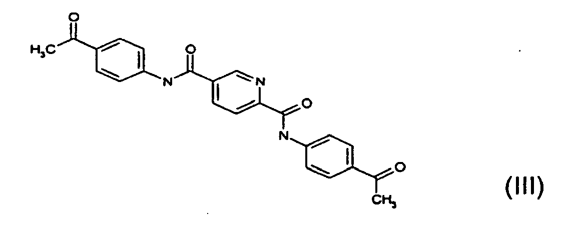

17. A compound according to claim 1 with formula IV:

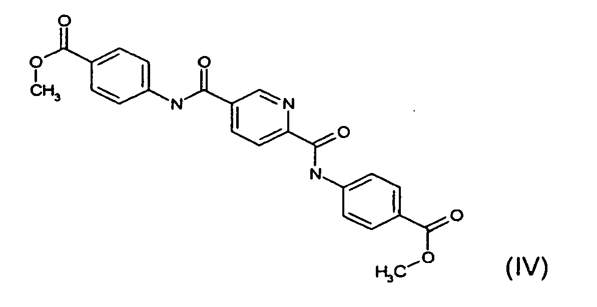

18. A compound according to claim 1 with formula V:

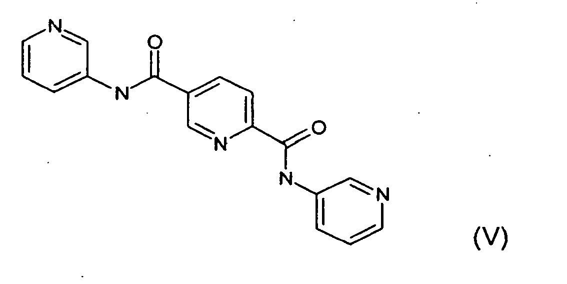

19. A compound according to claim 1 with formula VI:

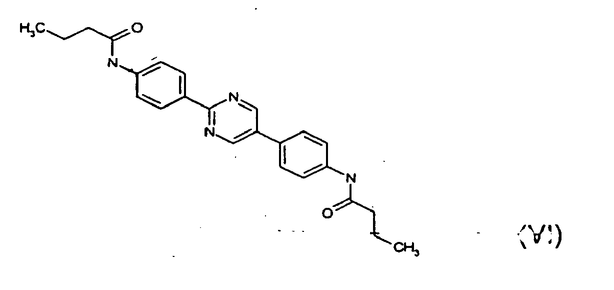

20. A compound according to claim 1 with formula VII:

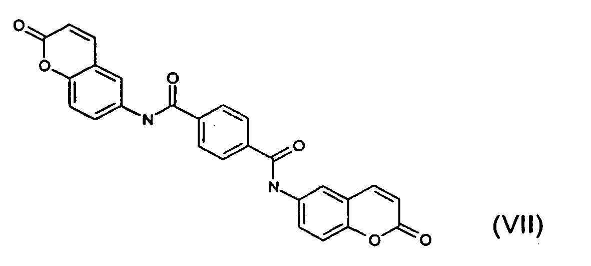

21. A compound according to claim 1 with formula VIII:

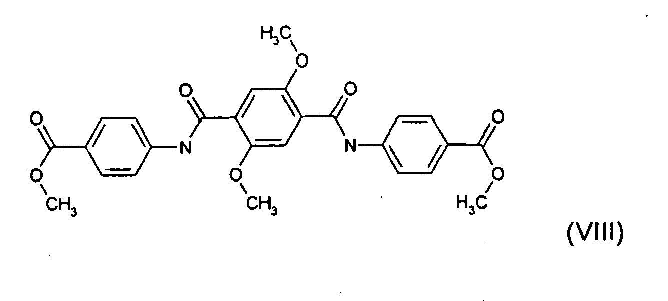

22. A compound according to claim 1 with formula IX:

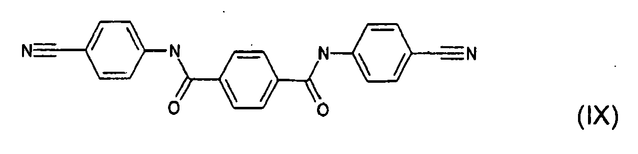

23. A compound according to claim 1 with formula X:

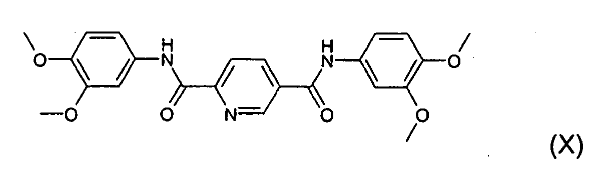

24. A compound according to claim 1 with formula XI:

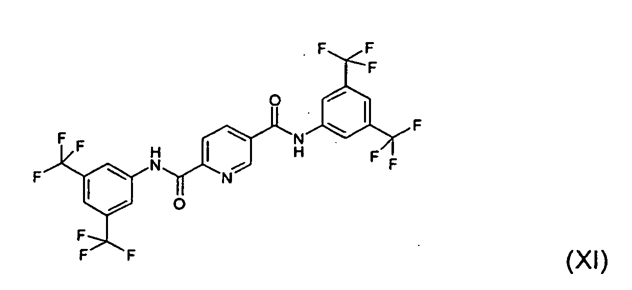

25. Use of a compound according to any one of the preceding claims as a medicament.

26. Use of a compound according to anyone of claims 1 to 24 for the manufacture of a pharmaceutical composition for the treatment of Wnt pathway dependent diseases.

27. Use of a compound according to anyone of the claims 1 to 24 for the manufacture of a pharmaceutical composition for the treatment of cancer.

28. Pharmaceutical composition comprising a compound according to anyone of the claims 1 to 24 or a pharmaceutically acceptable salt thereof.

29. Pharmaceutical composition according to claim 28, further comprising at least one pharmaceutically acceptable carrier, diluent or excipient.

## Patentansprüche

1. Verbindung der Formel I 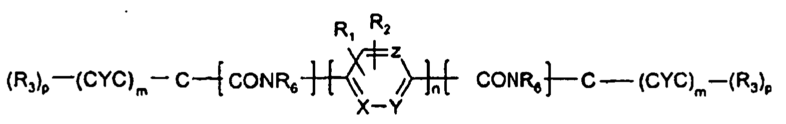 worin X, Y, Z unabhängig voneinander C oder N darstellen,
n für 1,2,3, steht, m 0 oder 1 ist, p für 0 oder eine ganze Zahl von 1 bis 6 steht, R₁, R₂ unabhängig voneinander Wasserstoff, ein Halogenatom, eine Hydroxylgruppe, eine C₁-C₃-Alkylgruppe und eine C₁-C₃-Alkoxygruppe darstellen,
R₃ unabhängig voneinander, falls p nicht 0 ist, Wasserstoff, Halogen, eine lineare oder verzweigte C₁-C₅-Alkyl-, eine Carboxyl-, eine Carbomethoxyl-, Carboethoxyl-, eine Benzyl-, eine Acyl-, eine Hydroxyl-, eine lineare oder verzweigte C₁-C₄-Alkoxyl-, eine Trifluormethyl-, eine Cyano-, eine Morpholino-, eine 1,3-Dioxolyl-, eine N-Acetylamidyl- oder eine Amidoylgruppe, einen gesättigten 5-8 gliedrigen Ring, einen heterocyclischen Ring, gegebenenfalls substituiert durch eine C₁-C₃-Alkyl-, eine Hydroxyl- oder eine Benzylgruppe, eine C₁-C₆-Alkylsulfonyl-, eine mono- oder disubstituierte C₁-C₅-Alkylgruppe, ein verzweigtes oder cyclisches Amin, darstellt,
R₆ H oder Teil eines alicyclischen oder heterocyclischen Ringsystems ist, unter der Bedingung dass,
wenn m 0 ist, dann C CF₃ oder eine verzweigte oder unverzweigte C₁-C₄-Alkylgruppe darstellt,
wenn m=1 ist, dann C -CH₂-O-, -CH₂-, -CH₂CH₂-, -CH₂CH₂CH₂-, - CH₂(CH₂)CH₂- darstellt oder eine chemische Bindung zwischen N-C oder C-C Atomen bedeutet,
die CONR₆-Gruppe entweder über ihr Kohlenstoff- oder ihr Stickstoffatom an C gebunden sein kann,
CYC für ein durch R₃ substituiertes Phenyl, Pyridinyl, Naphthyl, Chinolinyl, Isochinolinyl, Thiophenyl-, 1,3,4-Thiadiazolidinyl-, Furanyl, Tetrahydrochinolinyl, Tetrahydroisochinolinyl, Morpholinyl, Furanyl, Cyclohexenyl und Chromen-2-on-yl steht.

2. Verbindung nach Anspruch 1, worin n 1 ist.

3. Verbindung nach Anspruch 1, worin n 2 ist.

4. Verbindung nach Anspruch 1, worin n 3 ist.

5. Verbindung nach einem der Ansprüche 1 bis 4, worin X, Y und Z C sind.

6. Verbindung nach einem der Ansprüche 1 bis 4, worin X und Y C sind und Z N ist.

7. Verbindung nach einem der Ansprüche 1 bis 4, worin X C ist und Y und Z N sind.

8. Verbindung nach einem der Ansprüche 1 bis 7, worin m 0 ist.

9. Verbindung nach Anspruch 8, worin C CF₃ oder eine verzweigte oder unverzweigte C₁-C₄-Alkylgruppe ist.

10. Verbindung nach Anspruch 9, worin C tert-Butyl ist.

11. Verbindung nach einem der Ansprüche 1 bis 7, worin m 1 ist.

12. Verbindung nach Anspruch 11, worin C -CH₂-O-, -CH₂-, -CH₂CH₂-, - CH₂CH₂CH₂-, -CH₂(CH₂)CH₂- ist oder eine chemische Bindung zwischen N-C oder C-C Atomen bedeutet.

13. Verbindung nach einem der Ansprüche 1 bis 12, worin die CONR₆-Gruppe über ihr Kohlenstoffatom an C gebunden ist.

14. Verbindung nach einem der Ansprüche 1 bis 12, worin die CONR₆-Gruppe über ihr Stickstoffatom an C gebunden ist.

15. Verbindung nach Anspruch 1 der Formel II:

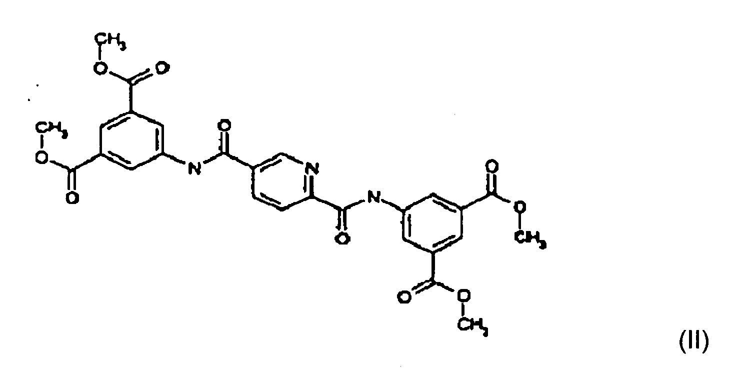

16. Verbindung nach Anspruch 1 der Formel III:

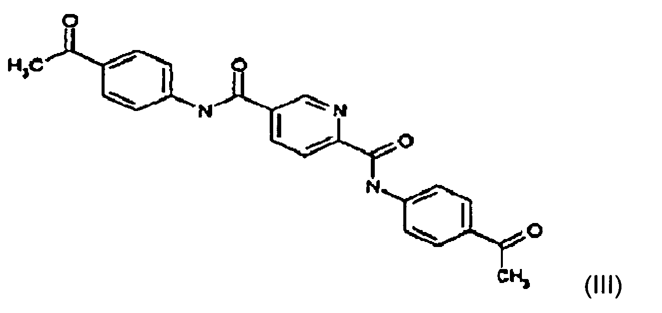

17. Verbindung nach Anspruch 1 der Formel IV:

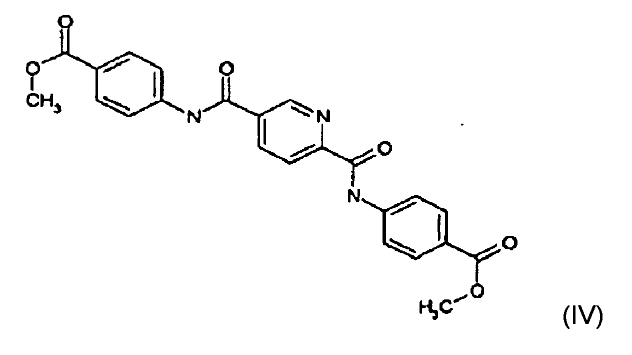

18. Verbindung nach Anspruch 1 der Formel V:

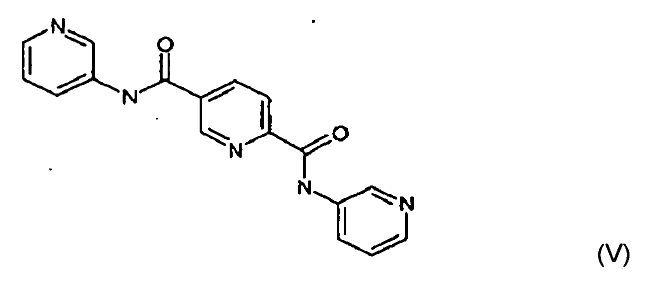

19. Verbindung nach Anspruch 1 der Formel VI:

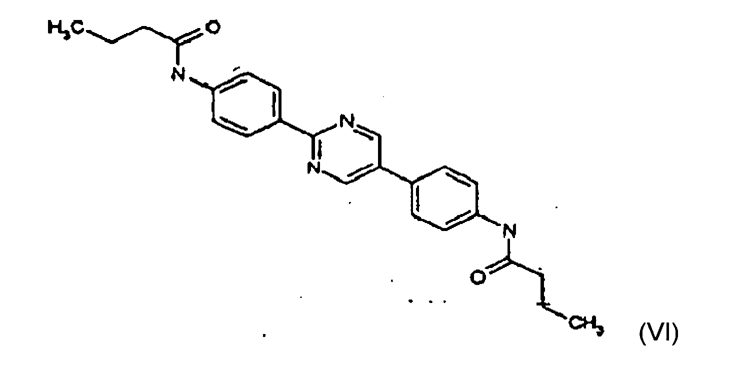

20. Verbindung nach Anspruch 1 der Formel VII:

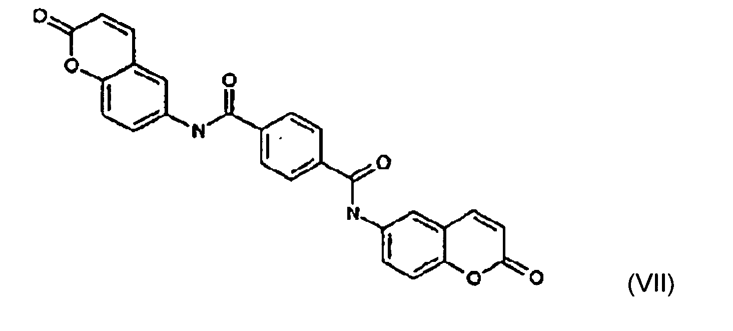

21. Verbindung nach Anspruch 1 der Formel VIII:

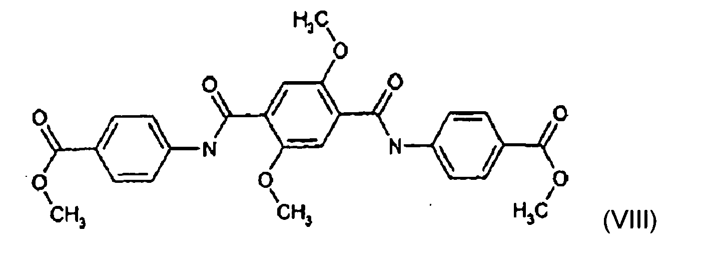

22. Verbindung nach Anspruch 1 der Formel IX:

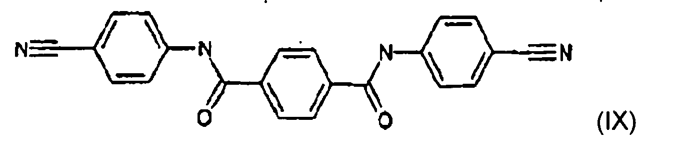

23. Verbindung nach Anspruch 1 der Formel X:

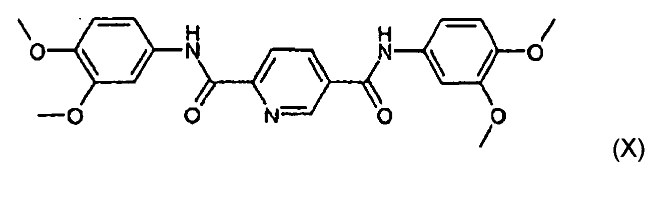

24. Verbindung nach Anspruch 1 der Formel XI:

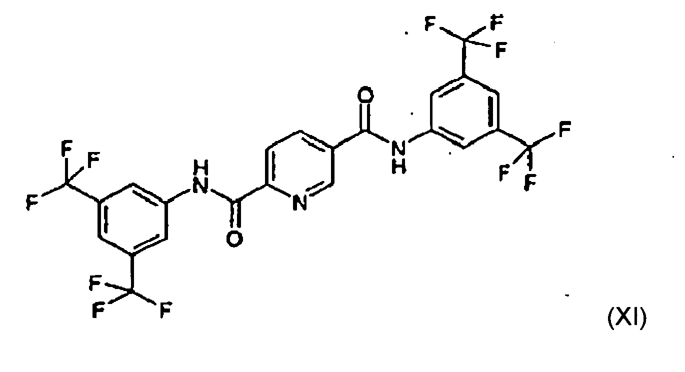

25. Verwendung einer Verbindung nach einem der vorhergehenden Ansprüche als Arzneimittel.

26. Verwendung einer Verbindung nach einem der Ansprüche 1 bis 24 zur Herstellung einer pharmazeutischen Zusammensetzung zur Behandlung von Erkrankungen, die vom Wnt Signalweg abhängen.

27. Verwendung einer Verbindung nach einem der Ansprüche 1 bis 24 zur Herstellung einer pharmazeutischen Zusammensetzung zur Behandlung von Krebs.

28. Pharmazeutische Zusammensetzung, umfassend eine Verbindung nach einem der Ansprüche 1 bis 24 oder ein pharmazeutisch verträgliches Salz davon.

29. Pharmazeutische Zusammensetzung nach Anspruch 28, weiterhin umfassend wenigstens einen pharmazeutisch verträglichen Träger, Verdünnungsmittel oder Hilfsstoff.

## Revendications

1. Composé de formule I 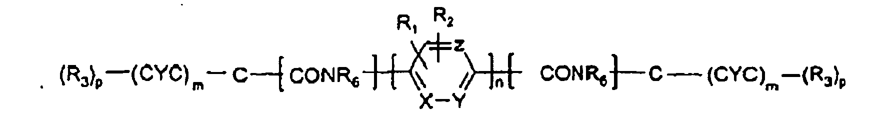 dans laquelle
X, Y, Z représentent indépendamment les uns des autres C ou N,
n représente 1, 2, 3, m est 0 ou 1, p représente 0 ou un entier de 1 à 6,
R₁, R₂ représentent indépendamment l'un de l'autre hydrogène, un atome d'halogène, un groupe hydroxyle, un groupe alkyle en C₁-C₃ et un groupe alcoxyle en C₁-C₃,
R₃ représente indépendamment l'un de l'autre si p n'est pas 0, hydrogène, halogène, un alkyle linéaire ou ramifié en C₁-C₅, un carboxylyle, un carbométhoxyle, un carboéthoxyle, un benzyle, un acyle, un hydroxyle, un alcoxyle linéaire ou ramifié en C₁-C₄, un trifluorométhyle, un cyano, un morpholino, un 1,3-dioxolyle, un groupe N-acétylamidyle ou amidoyle, un anneau saturé à 5 à 8 éléments, un anneau hétérocyclique, substitué en option par un groupe alkyle en C₁-C₃, hydroxyle ou benzyle, un alkylsulfonyle en C₁-C₆, un groupe alkyle en C₁-C₅ monosubstitué ou disubstitué, une amine ramifiée ou cyclique,
R₆ est H ou fait partie d'un système d'anneau alicyclique ou hétérocyclique,
avec la condition
si m est 0, alors C représente CF₃ ou un groupe alkyle en C₁-C₄ ramifié ou non ramifié,
si m est 1, alors C représente -CH₂-O-, -CH₂-, -CH₂CH₂-, -CH₂CH₂CH₂-, -CH₂(CH₂)CH₂- ou dénote une liaison chimique entre des atomes N-C ou C-C,
le groupe CONR₆ peut être lié à C soit par le biais de son atome de carbone, soit par le biais de son atome d'azote,
CYC représente un phényle, pyridinyle, naphtyle, quinolinyle, isoquinolinyle, isoxaxolinyle, thiophényle, 1,3,4-thiadiazazolidinyle, furanyle, tétrahydroquinolinyle, tétrahydroisoquinolinyle, morpholinyle, furanyle, cyclohexényle et chromén-2-on-yle substitué par R₃ ou non substitué.

2. Composé selon la revendication 1, dans lequel n est 1.

3. Composé selon la revendication 1, dans lequel n est 2.

4. Composé selon la revendication 1, dans lequel n est 3.

5. Composé selon l'une quelconque des revendications 1 à 4, dans lequel X, Y et Z sont C.

6. Composé selon l'une quelconque des revendications 1 à 4, dans lequel X et Y sont C et Z est N.

7. Composé selon l'une quelconque des revendications 1 à 4, dans lequel X est C et Y et Z sont N.

8. Composé selon l'une quelconque des revendications 1 à 7, dans lequel m est 0.

9. Composé selon la revendication 8, dans lequel C est CF₃ ou un groupe alkyle en C₁-C₄ ramifié ou non ramifié.

10. Composé selon la revendication 9, dans lequel C est tert-butyle.

11. Composé selon l'une quelconque des revendications 1 à 7, dans lequel m est 1.

12. Composé selon la revendication 11, dans lequel C est -CH₂-O-, -CH₂-, -CH₂CH₂-, -CH₂CH₂CH₂-, -CH₂(CH₂)CH₂-ou dénote une liaison chimique entre des atomes N-C ou C-C.

13. Composé selon l'une quelconque des revendications 1 à 12, dans lequel le groupe CONR₆ est lié à C par son atome de carbone.

14. Composé selon l'une quelconque des revendications 1 à 12, dans lequel le groupe CONR₆ est lié à C par son atome d'azote.

15. Composé selon la revendication 1 avec la formule II :

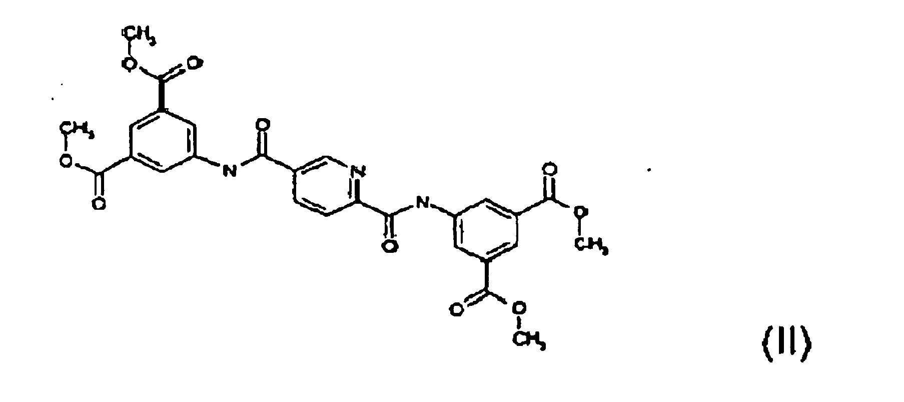

16. Composé selon la revendication 1 avec la formule III :

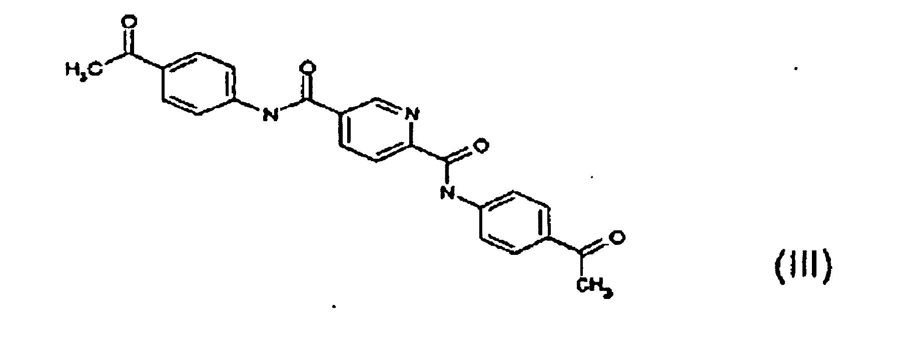

17. Composé selon la revendication 1 avec la formule IV :

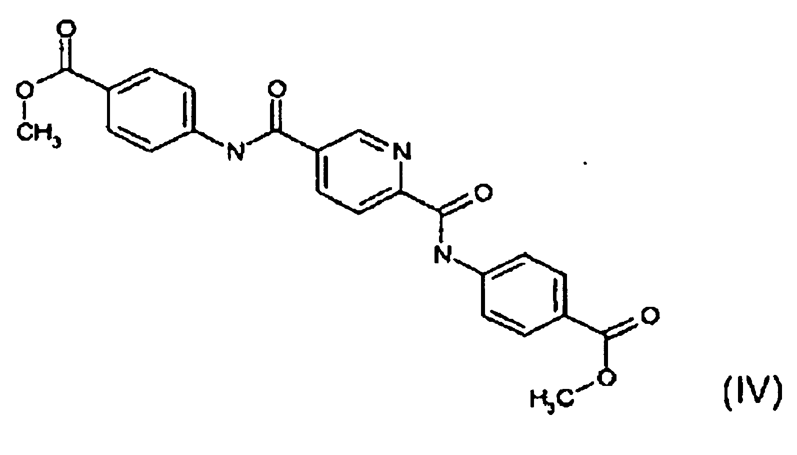

18. Composé selon la revendication 1 avec la formule V :

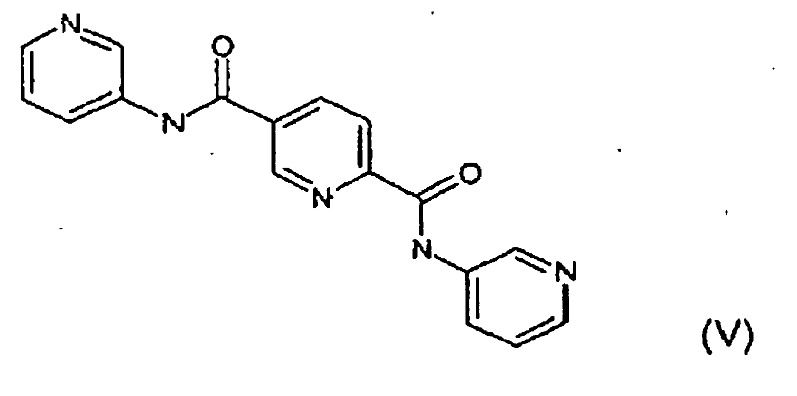

19. Composé selon la revendication 1 avec la formule VI :

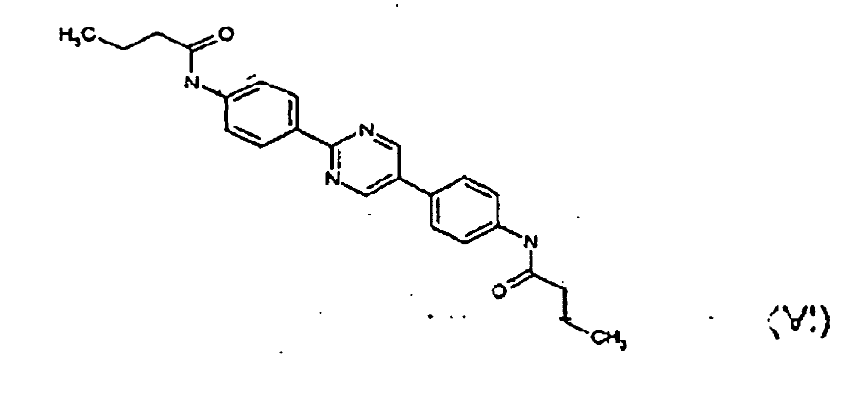

20. Composé selon la revendication 1 avec la formule VII :

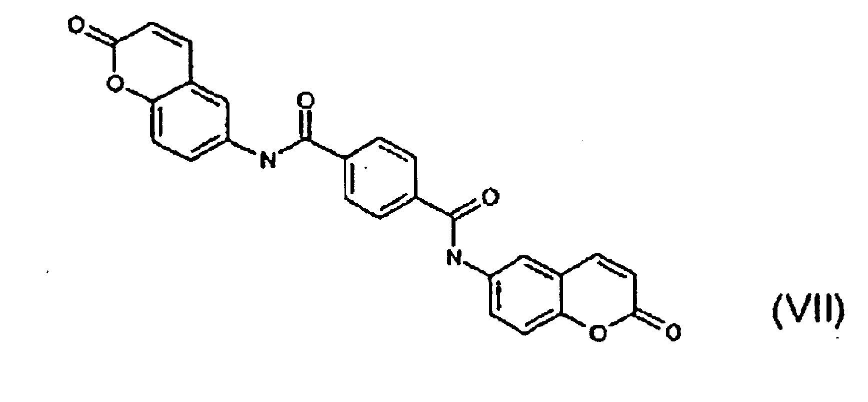

21. Composé selon la revendication 1 avec la formule VIII :

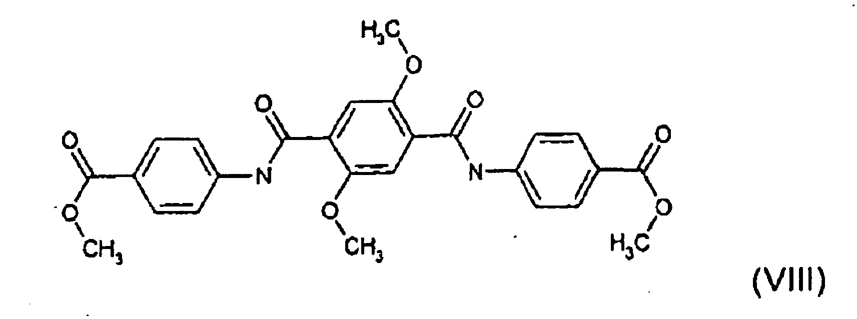

22. Composé selon la revendication 1 avec la formule IX :

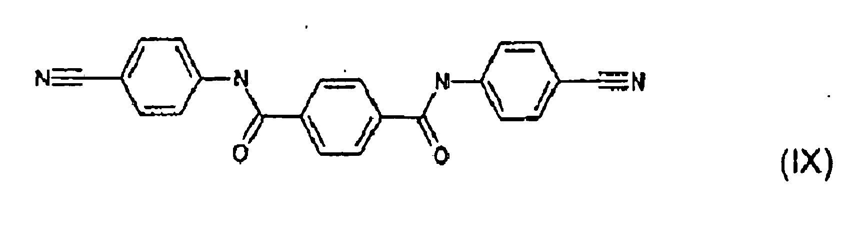

23. Composé selon la revendication 1 avec la formule X :

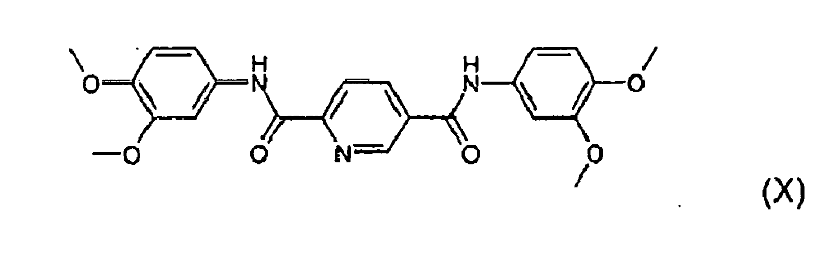

24. Composé selon la revendication 1 avec la formule XI :

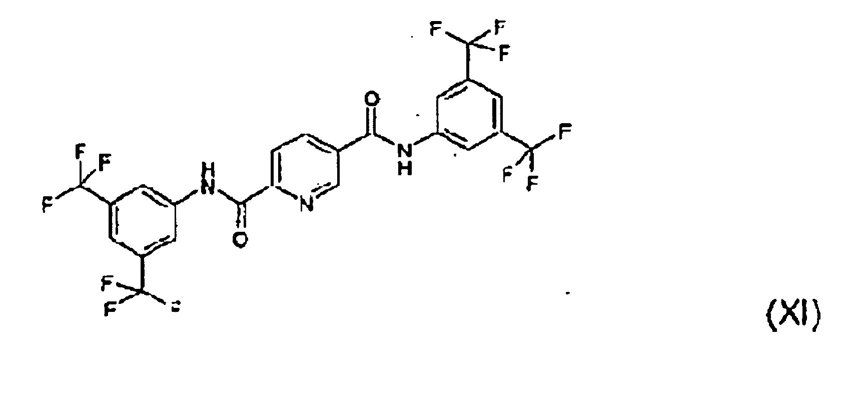

25. Utilisation d'un composé selon l'une quelconque des revendications précédentes en tant que médicament.

26. Utilisation d'un composé selon l'une quelconque des revendications 1 à 24 pour la fabrication d'une composition pharmaceutique pour le traitement de maladies dépendant de la voie Wnt.

27. Utilisation d'un composé selon l'une quelconque des revendications 1 à 24 pour la fabrication d'une composition pharmaceutique pour le traitement du cancer.

28. Composition pharmaceutique comprenant un composé selon l'une quelconque des revendications 1 à 24 ou un sel pharmaceutiquement acceptable de celui-ci.

29. Composition pharmaceutique selon la revendication 28, comprenant en outre au moins un support, diluant ou excipient pharmaceutiquement acceptable.
